# EUROPEAN PATENT APPLICATION

(11) **EP 3 603 532 A1**
(43) Date of publication of application: **05.02.2020**
(21) Application number: 18774356.2
(22) Date of filing: 30.03.2018
(51) Int. Cl.: A61B 17/00, A61N 7/00

(54) **ULTRASONIC IRRADIATION DEVICE**

(30) Priority: 30.03.2017 JP 2017069316
(71) Applicant: Kyocera Corporation, Kyoto-shi, Kyoto 612-8501 (JP)
(72) Inventor: TAKANO, Yasuju, Kyoto-shi Kyoto 612-8501 (JP)
(74) Representative: Viering, Jentschura & Partner mbB Patent- und Rechtsanwälte
(86) International application number: PCT/JP2018/013978
(87) International publication number: WO 2018/182017

(57) **Abstract**

An ultrasonic irradiation device according to the disclosure includes an ultrasonic transducer for external application of HIFU, which is focused ultrasound, to a body surface; and an optical imaging member or camera that takes images of part of the body surface corresponding to an ultrasonic irradiation target area. An irradiation position guide or a marker is located on the body surface, the marker is detected by the camera, and position and irradiation angle of the ultrasonic transducer are controlled based on information about a position of the marker. This makes it possible to direct a therapeutic transducer to a proper irradiation position, and monitor body surface condition during ultrasonic irradiation.

## Description

### Technical Field

The present disclosure relates to an ultrasonic irradiation device for external application of ultrasound waves to a painful body area for ablation.

### Background Art

As a way to alleviate ache and pain arising from metastatic bone cancer, painful joint, etc., there has been non-invasive bone-pain relief treatment to ablate a painful body area by external application of high-intensity focused ultrasound (hereafter referred to as "HIFU") to the affected area using an ultrasonic irradiation device (refer to Patent Literatures 1 and 2).

### Citation List

### Patent Literature

Patent Literature 1: Japanese Examined Patent Publication JP-B2 7-38858 (1995)
Patent Literature 2: Japanese Examined Patent Publication No. JP-B2 5429822

### Summary of Invention

An ultrasonic irradiation device according to the disclosure includes an ultrasonic transducer for external application of focused ultrasound to a body surface, and an optical imaging member that takes images of part of the body surface corresponding to an ultrasonic irradiation target area.

### Brief Description of Drawings

Other and further objects, features, and advantages of the invention will be more explicit from the following detailed description taken with reference to the drawings wherein:
FIG. 1 is a schematic drawing of an ultrasonic irradiation device according to the first embodiment;
FIG. 2 is a schematic drawing of an ultrasonic irradiation device according to the second embodiment;
FIGS. 3A to 3D are each a front view showing an example of an irradiation position guide used in the embodiments; and
FIG. 4A is a front view of an ultrasonic irradiation portion of the ultrasonic irradiation device according to the embodiments as seen from below, and FIG. 4B is a side view thereof.

### Description of Embodiments

Currently-used ultrasonic irradiation equipment designed for bone-pain relief treatment is classified into two types according to the manner of diagnosis and guide for an ultrasonic irradiation target area such as an affected area subjected to medical treatment, namely (1) an apparatus with an MRI (Magnetic Resonance Imaging)-guided system called "MRgHIFU" and (2) an apparatus with an ultrasound-guided system called "USgHIFU". Both of them employ a therapeutic HIFU transducer for ablation of painful areas. There are HIFU transducers of various shapes, including one having a circular or rectangular contour, the surface of which is curved concavely in a direction opposite to the direction of ultrasonic irradiation, and one having a plate-like form with no curved surface.

In each of the MRgHIFU and USgHIFU, an area of pain is identified with the aid of images obtained by the MRI-guided system or the ultrasound-guided system, and the therapeutic transducer is put on the identified area to effect HIFU irradiation for ablation of the affected area.

Unfortunately, currently-used typical diagnostic MRI-guided systems and ultrasound-guided systems, while being capable of detection and imaging of organs such as soft tissue deep inside human body that is responsible for deep-aching pain, do not lend themselves to detection and identification of bone pain-causing hard tissue in a superficial part of human body such as a region from the body surface to a depth of about a few to a few tens of millimeters below the surface.

Under the circumstances, to identify the position of a bone pain-affected area at a depth of about a few millimeters to 20 mm below the body surface, a doctor has to locate a point of tenderness on pressure by manual palpation, and then put a marking on part of the skin of the body surface which corresponds to the tenderness point with a pen for medical use, for example. With the aid of the marking, the doctor or other medical professional carries out positioning of the therapeutic transducer for HIFU irradiation by manual operation with visual estimation. That is, there is no means for checking whether the therapeutic transducer is set in a proper irradiation position.

Furthermore, in the event of heat-caused-injury or burn in the skin due to ultrasonic irradiation and ultrasonic ablation, although the MRI-guided system and ultrasound-guided system serve to examine the extent of the skin injury after ultrasonic irradiation, they are not capable of effecting imaging operation and measurement operation simultaneously during ultrasonic irradiation. That is, the conventional ultrasonic irradiation device is devoid of means for carrying out real-time monitoring of a condition of a skin during ultrasonic irradiation.

The disclosure has been made in view of the circumstances as discussed supra, and accordingly its object is to provide an ultrasonic irradiation device that directs a therapeutic transducer to a proper irradiation position, and monitors body surface condition during ultrasonic irradiation.

The following describes an embodiment of the disclosure.

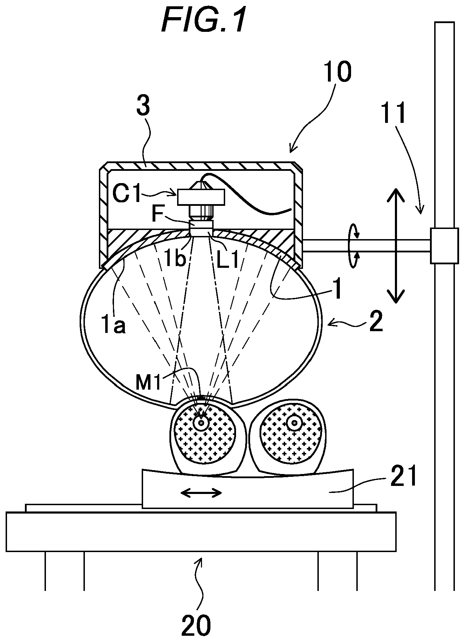

An ultrasonic irradiation device according to this embodiment is an ultrasonic irradiation device designed for bone-pain relief treatment, which is used to alleviate bone pain from metastatic bone cancer, painful joint, etc., in other words, ache and pain occurring at moderate depths ranging from about a few millimeters to 30 mm below the body surface. The ultrasonic irradiation device includes: an ultrasonic transducer for external application of focused ultrasound to a body surface; and an optical imaging member that takes images of part of the body surface corresponding to an ultrasonic irradiation target area. As shown in FIG. 1, the ultrasonic irradiation device includes a HIFU irradiation section 10 including a single ultrasound emitter or a plurality of ultrasound emitters and a treatment support table 20, and further includes a power supply section, a coolant producing section, and a control section such as a personal computer, which are not shown.

A movable bed 21 on the treatment support table 20 and a support 11 for supporting the HIFU irradiation section 10 are movable in the directions of two plane coordinate axes, namely X-axis direction and Y-axis direction, and in the direction of a height coordinate axis, namely Z-axis direction, respectively, so that the position of patient's body lying in the bed under ultrasonic irradiation treatment can be freely adjusted. This makes it possible to hold an ultrasonic irradiation target area, such as the thighs of both legs of the patient in this case, in a proper position immediately below the HIFU irradiation section 10 in a vertical direction.

The HIFU irradiation section 10 may be made rotatable about the axis of the support 11 so as to apply ultrasound waves obliquely relative to the vertical direction. Moreover, the movable bed 21 may be equipped with, for example, a holding strap for retaining the posture and position of the patient. Mechanisms for moving and holding part of patient's body subjected to ultrasonic irradiation are not limited to those as described above, and, it is possible to use any heretofore known patient-supporting and moving mechanism that is adopted for use in other medical testing equipment such as X-ray examination equipment on account of its capability of holding an ultrasonic irradiation target area in a proper position.

In the ultrasonic irradiation device according to this embodiment, the ultrasonic transducer includes an ultrasonic irradiation portion having a surface opposed to an irradiation target. The ultrasonic irradiation portion has an opening formed in its surface opposed to the irradiation target, and, the optical imaging member is located for imaging of the part of the body surface corresponding to the ultrasonic irradiation target area through the opening.

More specifically, the HIFU irradiation section 10 as shown in FIG. 1 mainly includes: an ultrasonic transducer 1, which serves as an ultrasonic irradiation portion, shaped in a circular plate so as to spread out above the affected area; a resin film-made balloon 2 that receives therein cooling water for cooling part of patient's body subjected to ultrasonic irradiation which is the ultrasonic irradiation target area; and a housing 3 built as an irradiation portion case that enables the ultrasonic transducer 1 and the balloon 2 to be suspended above patient's body, and is movably supported by the support 11. Means for providing connection between the constituent members, such as piping and wiring, is not shown in each drawing.

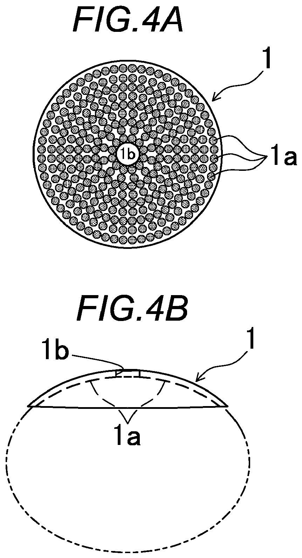

As shown in FIG. 4B, the ultrasonic transducer 1 is in the general form of a circular plate or a disk shaped so as to spread out above the affected area. The lower surface of the ultrasonic transducer 1 facing toward the affected area is curved concavely in a vertical downwards direction to define a parabolic surface. As shown in FIG. 4A, on the inner side of the concave surface, there are provided many ultrasound emitters 1a. That is, the inner surface of the ultrasonic transducer 1 is curved with a radius of curvature such that ultrasound waves can converge to a point. Thus, when the individual ultrasound emitters 1a oscillate simultaneously, as shown in FIG. 1, resultant ultrasound emission is focused to one point, with consequent production of high-intensity focused ultrasound, viz., HIFU.

Moreover, the center of the discotic and parabolic form is provided with a through hole 1b as an opening capable of mounting a lens of a camera C1 which serves as a first optical imaging element as described later. Based on information about the detection of a marker M1 serving as a guide in identifying an irradiation position located below the balloon 2, and its position obtained by the camera C1 mounted in the through hole 1b, the control section such as a computer, which are not shown, effects control of the HIFU irradiation section 10 including the ultrasonic transducer 1 and the balloon 2 in such a manner as to move downward and stay in a proper position immediately above the marker M1.

The ultrasonic transducer 1 is, as exemplified, shaped in a circular plate or the like so as to spread out above the affected area. This is intended to arrange as many ultrasound emitters as possible on a wide surface opposed to the affected area to ensure high-intensity ultrasound emission. Thus, the general form of the ultrasonic irradiation portion is not limited to the circular plate or disk, and it is possible to use any other shape having a surface wide enough to face toward the affected area without special limitation. For example, the ultrasonic irradiation portion may be given a circular or rectangular contour, the surface of which is curved concavely in a direction opposite to the direction of ultrasonic irradiation, or may be shaped in a plate having no curve.

Moreover, as the ultrasound emitters, phased-array type HIFU emitters capable of adjusting an ultrasound focusing point and a depth of field may be used. This makes it possible to change the radius of curvature of the parabolic form of the ultrasonic transducer 1, as well as to adopt a flat-shaped irradiation portion having a flat lower surface.

The balloon 2 is constructed of a resin-made transparent or semi-transparent film. Degassed cold water produced by the coolant producing section, which is not shown, such as cold water of about 18°C in this case, is passed in circulation into the balloon 2 from the coolant producing section. This makes it possible to keep water temperature in the balloon 2 constant, and thereby cool the body surface contacted by the bottom of the lower part of the balloon 2.

The reason for cooling the skin surface of the ultrasonic irradiation target area is to reduce heat-caused-injury or burn in the body surface and body's superficial part under ultrasonic irradiation. The cooling water is circulated in a degassed state to reduce generation of air bubbles due to ultrasonic irradiation, for example. Moreover, the "transparent or semi-transparent" film used for the balloon 2 according to this embodiment refers to a resin film whose transmissivity for light with wavelengths in the visible and ultraviolet regions is greater than or equal to 80%.

As described earlier, the camera C1 is mounted in the through hole 1b formed centrally of the discotic ultrasonic transducer 1. With the camera C1, part of the body surface corresponding to the ultrasonic irradiation target area located immediately below the balloon 2 can be imaged through the cooling water received in the balloon 2 and the resin-made film constituting the lower part of the balloon 2. Moreover, the tip of the lens-side part, or equivalently the lower end as viewed in the drawing, of the camera C1 is provided with an optical filter F and a light L1 facing toward the interior of the balloon 2. This allows clearer imaging of the ultrasonic irradiation target area marked with the marker M1.

A sealing member such as an O ring is disposed as caulking means for watertightness between the optical filter F, as well as the light L1, mounted at the tip of the camera C1 and the surrounding through hole 1b to prevent leakage of the cooling water. The water leakage through the through hole 1b may be prevented by other means.

In the ultrasonic irradiation device thereby constructed, the therapeutic HIFU irradiation section 10 can be guided to a proper irradiation position with ease by utilizing analytical data on an image of part of the body surface corresponding to the ultrasonic irradiation target area taken by the camera C1, and, body surface condition at the ultrasonic irradiation target area can be monitored on a real-time basis during ultrasonic irradiation.

That is, in the ultrasonic irradiation device according to this embodiment, the therapeutic transducer can be guided to a proper irradiation position with ease by utilizing analytical data on an image of part of the body surface corresponding to the ultrasonic irradiation target area taken by the optical imaging member, and, a condition of a skin at part of the body surface corresponding to the ultrasonic irradiation target area can be observed in a monitor on a real-time basis during ultrasonic irradiation. Thus, the ultrasonic irradiation device according to this embodiment reduces the burdens on patients under ultrasonic irradiation treatment.

Moreover, the ultrasonic irradiation device according to this embodiment effects imaging of an ultrasonic irradiation target area, which is hidden immediately below the ultrasonic irradiation portion having substantially the form of a circular plate or a disk shaped so as to spread out above the affected area, during ultrasonic irradiation without the necessity of moving the therapeutic HIFU transducer. This makes it possible to reduce, for example, time spent on ultrasonic irradiation treatment, and thereby reduce the burdens on patients.

The monitoring of an ultrasonic irradiation target area will be described in more detail. In this embodiment, the capability of imaging the irradiation target area during ultrasonic irradiation comes from designing the balloon 2 to have a transparent or semi-transparent bottom, from using transparent or semi-transparent liquid as the degassed water received in the balloon 2, and from positioning the camera C1 so that it can find the location of the marker M1 indicating the ultrasonic irradiation target area hidden below the ultrasonic irradiation portion through the interior of the balloon 2. Moreover, the camera C1 includes the optical filter F and the light L1, and is thus able to take images in various imaging modes.

The ultrasonic irradiation device according to this embodiment includes an image processing section that carries out computation on image data obtained by the optical imaging member. The image processing section includes a skin condition determination portion that determines a condition of a skin at the ultrasonic irradiation target area based on the distribution of reflection of light in a plurality of specific wavelength regions from the ultrasonic irradiation target area in the obtained image.

For example, in monitoring body surface condition with attention given to an image of reflected light from the ultrasonic irradiation target area, with use of standard illuminant A in the form of a tungsten lamp intended to represent "typical lighting" with a color temperature of 2856.6 K, or standard illuminant D₆₅ intended to represent daylight with a correlated color temperature of 6504 K as the light L1, the location of the marker M1 observable through the balloon 2 is imaged by the camera C1. There is thus obtained an image of reflected light of wavelengths in the visible region ranging from about 400 nm to 700 nm. Moreover, the optical spectrum of the ultrasonic irradiation target area can be obtained by subjecting the obtained image to spectroscopic measurement at 10-nm intervals with the image processing section, not shown. Based on this optical spectrum, L* value, a* value, and b* value, which are color parameters in accordance with the CIELAB (L*a*b*) color space, can be determined by calculation.

Then, in respect of color parameters related to heat-caused-injury or burn in the skin surface, in particular, a red index at the a* axis and a yellow index at the b* axis, a comparison is drawn between an initial value before ultrasonic irradiation and a measured value during or after ultrasonic irradiation. Thus, a condition of a skin at the ultrasonic irradiation target area can be monitored on a real-time basis. Note that the foregoing is given as exemplification of the skin condition determination portion, and, the value a* may be utilized to assess the risk of heat-caused-injury in the skin of the ultrasonic irradiation target area.

According to the L*a*b* color space, in the 256-level a* scale extending from ao (fully saturated green) to a₂₅₅ (fully saturated red), a red index is represented by the a* values that are numbered from 128 to 255. Moreover, in the 256-level b* scale extending from bo (fully saturated blue) to b₂₅₅ (fully saturated yellow), a yellow index is represented by the b* values that are numbered from 128 to 255. With a record of initial a*, b* values for the target area yet to be subjected to ultrasonic irradiation, it is possible to obtain measured a*, b* values for the target area by carrying out similar imaging operation during ultrasonic irradiation or on other occasions without the necessity of moving the HIFU irradiation section 10. Then, the difference between the initial value and the measured value is determined as Delta to yield a clear understanding of changes in body surface color, in other words, changes in a condition of a skin.

Likewise, changes in a condition of a skin can be monitored from a different standpoint with attention given to reflectivity for light of a specific wavelength or wavelengths in a specific wavelength region in the reflection light spectrum, absorbency at a specific wavelength, etc.

That is, in the ultrasonic irradiation device according to this embodiment, the image processing section includes a heat-caused-injury risk assessment portion that assesses the risk of heat-caused-injury in the skin of the ultrasonic irradiation target area based on the distribution of reflection of light in a plurality of specific wavelength regions from the ultrasonic irradiation target area in the obtained image.

For example, a condition of a skin at the ultrasonic irradiation target area and the risk of heat-caused-injury may be determined by any of a method of assessing a degree of burn, or the extent of heat-caused-injury in the skin of the target area based on the distribution of reflection of light in a plurality of specific wavelength regions, such as light of wavelengths in the vicinity of 450 nm absorbed by the surface layer of the upper part of the skin or light of wavelengths in the vicinity of 550 nm and 650 nm absorbed by hemoglobin, a method of assessing the risk of heat-caused-injury or necrosis in the skin of the target area based on the distribution of reflection of light in a frequency range of about 500 nm to 600 nm which is absorbed by oxy-hemoglobin (HbO₂) and deoxy-hemoglobin (Hb) in the blood, and a method of assessing the extent of injury in the upper layer of the skin at the target area based on the distribution of reflection of light in the vicinity of 685 nm from skin collagen. Note that the foregoing is given to exemplify the skin condition determination portion or the heat-caused-injury risk assessment portion.

On the other hand, in monitoring body surface condition with attention given to fluorescence from the ultrasonic irradiation target area, with use of a diode such as laser LED or ultraviolet (UV) LED or a lamp for emitting excitation light of a specific wavelength as a light source such as the light L1, the location of the marker M1 is imaged by the camera C1. Thus, an image of fluorescence from the target area and fluorescence spectrum data can be obtained.

For example, an image of fluorescence including reflected light of wavelengths in the visible region ranging from about 450 nm to 700 nm is obtained through the application of light of a wavelength of about 405 nm from laser LED as a light source. In this case, occurrence of poor blood circulation resulting from heat-caused-injury is believed to lead to a phenomenon in which fluorescence of wavelengths ranging from about 500 nm to 550 nm from hemoglobin undergoes a decrease in light intensity. This allows an assessment as to the extent of heat-caused-injury in the skin of the target area.

Fluorescence comes also from skin collagen. For example, an image of fluorescence of wavelengths in the visible region ranging from about 400 nm to 410 nm is obtained through the application of light of a wavelength of about 325 nm from UV-LED. In this case, occurrence of skin damage resulting from heat-caused-injury is believed to lead to a phenomenon in which fluorescence of wavelengths of about 400 nm and 405 nm from collagen undergoes a decrease in light intensity. This allows an assessment as to the extent of heat-caused-injury in the skin of the target area, too. Note also that the foregoing is given to exemplify the skin condition determination portion or the heat-caused-injury risk assessment portion in this embodiment.

As practiced in the above-described embodiment, in the ultrasonic irradiation device according to the disclosure, with utilization of analytical data on an image of part of the body surface corresponding to an ultrasonic irradiation target area taken by the camera C1, body surface condition at the target area can be monitored on a real-time basis without the necessity of moving the HIFU irradiation section 10.

The control section of the ultrasonic irradiation device according to this embodiment may be provided with a heat-caused-injury preventive portion that stops ultrasound emission from the ultrasonic transducer 1 in a case where predetermined threshold levels of a condition of a skin and heat-caused-injury risk such for example as a* values obtained by the aforestated methods are exceeded. That is, by designing the skin condition determination portion or heat-caused-injury risk assessment portion to be operatively associated with the control section for controlling the ultrasonic transducer 1, it is possible to reduce occurrence of damage such as heat-caused-injury or burn more reliably.

The following describes an irradiation position guide used to guide the therapeutic HIFU irradiation section 10 to a proper irradiation position.

The ultrasonic irradiation device according to this embodiment includes an irradiation position guide for ultrasonic irradiation, the irradiation position guide being located on the body surface, and an ultrasonic irradiation position guiding portion, the irradiation position guide being detected by the optical imaging member, and position and irradiation angle of the ultrasonic transducer being controlled based on information about a position of the irradiation position guide. The aforenamed optical imaging member may either be identical with or differ from the earlier-described "optical imaging member that takes images of the part of the body surface corresponding to the ultrasonic irradiation target area", and, the function of the optical imaging member is not limited to "the capability of taking images of the part of the body surface corresponding to the ultrasonic irradiation target area through the ultrasonic irradiation portion". Moreover, the optical imaging member may be disposed at any given place, and, any desired number of optical imaging members may be provided.

The irradiation position guide serves as an index or a mark for guiding the HIFU irradiation section 10 built as a therapeutic transducer to a proper position for irradiation. The position of placement of the irradiation position guide is determined based on a point of tenderness detected on manual palpation by a doctor, for example.

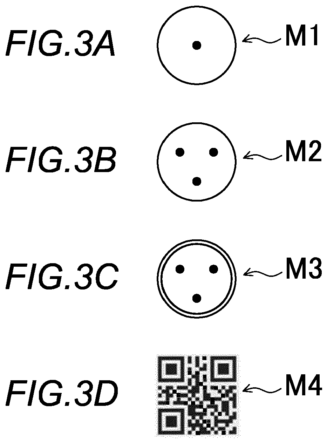

The irradiation position guide, for example, as shown in FIG. 3, any of film markers including the exemplified marker M1, or, a mark, a stamp, a sign, or the like printed in ink on part of the body surface corresponding to the ultrasonic irradiation target area, is disposed on the body surface.

That is, it is preferable that the ultrasonic irradiation device according to this embodiment employs a design wherein the irradiation position guide has the form of a film marker disposed on the body surface, or a design wherein the irradiation position guide has the form of a mark, a stamp, or a sign printed in ink on the body surface. The "print-marking on the body surface" is conceptual means encompassing various ways to put a marking on the skin, including printing of a marking using a printer or the like, writing of a marking by hand using a pen or the like, imprinting of a marking using a stamp or the like, and adhesion of a sticky marking.

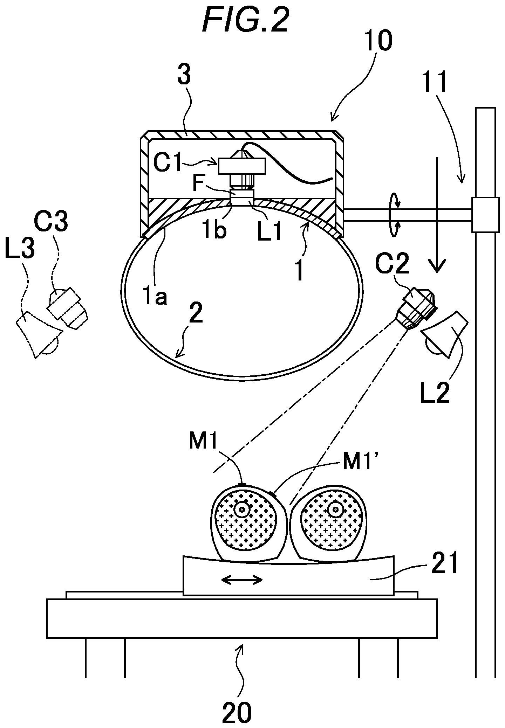

A plurality of irradiation position guides including the marker may be provided without special limitation as to number and placement position. Moreover, as part of the ultrasonic irradiation position guiding portion and the optical imaging member for detecting the irradiation position guide, not only the camera C1 disposed within the discotic ultrasonic transducer 1 as described earlier, but also a plurality of components such for example as a second camera C2 and a third camera C3 as shown in FIG. 2 may be disposed at positions capable of observation and detection of the irradiation position guide without special limitation as to placement position.

Moreover, in the ultrasonic irradiation device shown in FIG. 2, for example, illuminating portions L2 and L3 that illuminate a location where the irradiation position guide lies are provided for increased visibility of the irradiation position guide. With use of a UV lamp capable of ultraviolet radiation, the so-called black light, for the light L1, the illuminating portions L2 and L3, etc. for illuminating the irradiation position guide, and, with use of ultraviolet reflective ink, called fluorescent ink, for the ink used for the marker or print on the body surface, the irradiation position guide can be identified more clearly irrespective of whether it is illuminated indoors or not.

The film marker used as the irradiation position guide is formed of a thin and soft material, such as a resin film, so that it can adhere firmly to the skin when adhesively or otherwise bonded to the body surface without causing the entry of air into between the body surface and the marker. Specific preferred examples of resin in use include a polymeric material such as silicone that undergoes neither ultrasound absorption nor ultrasound reflection and is yet transmissive to ultrasound waves. Moreover, the thickness of the film is preferably equal to a quarter of ultrasound wavelength. Given that the frequency of ultrasound radiation is 1 MHz, then the thickness of the film is preferably set at about 370 µm. It is possible to use a transparent or semi-transparent film having light transmissivity of greater than or equal to 80% to enable the camera C1 to find the skin located below the film marker through the film.

For example, the film marker has a circular plan configuration which is about 5 to 30 mm in diameter as shown in FIGS. 3A to 3D, or a polygonal plan configuration, when viewed from a medical professional or when looked down on from the upper optical imaging member. As seen from the drawings, a print is made in ink or the like on the surface (front side) of the marker.

The print may be made in various colors and patterns without special limitation. For example, it is possible to use a marker M1 printed only with a centrally located black spot indicative of a point of tenderness as shown in FIG. 3A, a marker M2 printed with three spots arranged isometrically and equidistantly so as to surround a point of tenderness as shown in FIG. 3B, a marker M3 rimmed with a circular safety frame representing an irradiation limiting line as shown in FIG. 3C, and a marker M4 functions as a two-dimensional code as shown in FIG. 3D or a one-dimensional code for patient identification.

Also in the case of using a mark, a stamp, a sign, or the like printed in ink on the body surface by a doctor or other medical professional as the irradiation position guide, in a like manner, a print such as a mark or a stamp as shown in FIGS. 3A to 3D may be made on the skin with a seal, for example. Another sign such as a simple dot, a circle, or a cross may be used instead. In this case, a plurality of such signs are printed in a pattern surrounding a point of tenderness, and, the barycenter of the pattern is determined as a reference point in image analysis.

When the ink for use in printing constitutes an obstruction to observation and monitoring of the body surface by the camera C1 described earlier, invisible ink such as UV reflective ink may be used for printing.

When the pain is so acute that a touch on part of the body which corresponds to a point of tenderness has to be avoided, that is; when it is difficult to place the film marker, mark, stamp, sign, or the like on or around the affected area with a point of tenderness, like a marker M1' as shown in FIG. 2, a marker, a mark, a stamp, a sign, or the like may be placed in a location spaced slightly away from the tenderness point. In this case, the therapeutic HIFU irradiation section 10 is guided to an irradiation position with reference to the marker, etc.

In the ultrasonic irradiation device thereby constructed, with reference to the film marker, or a mark, a stamp, a sign, or the like serving as the irradiation position guide on the body surface, patient's body lying in a supine position on the movable bed 21 of the treatment support table 20 can be automatically moved to a proper position for irradiation by making adjustment to the positions of the movable bed 21 and the HIFU irradiation section 10 moving in the three-axis directions, and, the HIFU irradiation section 10 can be lowered, with the top of its main irradiation portion focused on a proper location.

Thus, the ultrasonic irradiation device according to this embodiment can reduce ultrasonic irradiation time required for ultrasonic ablation, and can reduce the burdens on patients accordingly. Besides, the ultrasonic irradiation device can prevent or reduce occurrence of heat-caused-injury in the skin due to ultrasonic ablation.

As the camera serving as the optical imaging member in the ultrasonic irradiation device according to this embodiment, for example, it is possible to use a digital camera for capturing still images and moving images constructed of can be made of a charge coupled device (CCD) image sensor, a complementary metal-oxide semiconductor (CMOS) image sensor, a lens, a memory, etc. The camera is equipped with a cutoff filter, a band-pass filter, etc. on an as needed basis.

Moreover, the constituent components as set forth in the description of the embodiment intended for ultrasound emission control, control of patient support and body movement, image processing operation, skin condition determination, heat-caused-injury risk assessment, and heat-caused-injury prevention, respectively, may be made in the form of software programs or applications that constitutes portions of the control section such as a computer, and designed to be operatively interconnected to one another.

No special structural limitation is imposed on the constituent components of the ultrasonic irradiation device according to this embodiment, except for the HIFU irradiation section 10, and, the disclosure is applicable not only to HIFU irradiation system-equipped ultrasonic irradiation devices but also to other wide-ranging like apparatuses.

Moreover, the disclosure may be embodied in other specific forms without departing from the spirit or essential characteristics thereof. The present embodiments are therefore to be considered in all respects as illustrative and not restrictive, the scope of the disclosure being indicated by the appended claims rather than by the foregoing description and all changes which come within the meaning and the range of equivalency of the claims are therefore intended to be embraced therein.

### Reference Signs List

- 1:: Ultrasonic transducer
1a: Ultrasound emitter
1b: Through hole
- 2:: Balloon
- 3:: Housing
- 10:: HIFU irradiation section
- 11:: Support
- 20:: Treatment support table
- 21:: Movable bed
- C1:: Camera
- C2, C3:: Camera
- L1:: Light
- L2, L3:: Illuminating portion
- M1, M1':: Marker
- M2, M3, M4:: Marker

## Claims

1. An ultrasonic irradiation device, comprising:
an ultrasonic transducer for external application of focused ultrasound to a body surface; and
an optical imaging member that takes images of part of the body surface corresponding to an ultrasonic irradiation target area.

2. The ultrasonic irradiation device according to claim 1, wherein the ultrasonic transducer comprises an ultrasonic irradiation portion comprising a surface opposed to an irradiation target,
the ultrasonic irradiation portion comprises an opening provided in the surface opposed to the irradiation target, and
the optical imaging member is located so that the part of the body surface corresponding to the ultrasonic irradiation target area can be imaged through the opening.

3. The ultrasonic irradiation device according to claim 1 or 2, further comprising an image processing section that carries out computation on an image obtained by the optical imaging member,
wherein the image processing section comprises a skin condition determination portion that determines a condition of a skin at the ultrasonic irradiation target area based on a distribution of reflection of light in a plurality of specific wavelength regions from the ultrasonic irradiation target area in the obtained image.

4. The ultrasonic irradiation device according to claim 3, wherein the image processing section comprises a heat-caused-injury risk assessment portion that assesses a risk of heat-caused-injury in the skin of the ultrasonic irradiation target area based on a distribution of reflection of light in a plurality of specific wavelength regions from the ultrasonic irradiation target area in the obtained image.

5. The ultrasonic irradiation device according to claim 4, further comprising a heat-caused-injury preventive portion that stops ultrasound emission from the ultrasonic transducer in a case where a predetermined threshold level of the risk obtained by the heat-caused-injury risk assessment portion is exceeded.

6. The ultrasonic irradiation device according to claim 1, further comprising an irradiation position guide for ultrasonic irradiation,
the irradiation position guide being located on the body surface; and
an ultrasonic irradiation position guiding portion,
the irradiation position guide being detected by the optical imaging member, and position and irradiation angle of the ultrasonic transducer being controlled based on information about a position of the irradiation position guide.

7. The ultrasonic irradiation device according to claim 6, wherein the irradiation position guide includes a film marker disposed on the body surface.

8. The ultrasonic irradiation device according to claim 6, wherein the irradiation position guide includes a mark, a stamp, or a sign printed in ink on the body surface.

9. The ultrasonic irradiation device according to claim 6, wherein the irradiation position guide includes a one- or two-dimensional code for patient identification.

10. The ultrasonic irradiation device according to any one of claims 6 to 9, further comprising an illuminating portion that illuminates the irradiation position guide.
